(19) Europäisches Patentamt
European Patent Office
Office européen des brevets

(11) Veröffentlichungsnummer: **0 025 944**
**A1**

# (12) EUROPÄISCHE PATENTANMELDUNG

(21) Anmeldenummer: **80105425.5**

(51) Int. Cl.³: **C 07 C 149/12**

(22) Anmeldetag: **11.09.80**

(30) Priorität: **21.09.79 DE 2938156**

(43) Veröffentlichungstag der Anmeldung:
**01.04.81 Patentblatt 81/13**

(84) Benannte Vertragsstaaten:
**BE DE FR GB IT NL**

(71) Anmelder: **RHEIN-CHEMIE RHEINAU GMBH**
**Postfach 81 04 09**
**D-6800 Mannheim 81(DE)**

(72) Erfinder: **Sauerbier, Michael, Dr.**
**Promenadeweg 36**
**D-6831 Brühl(DE)**

(72) Erfinder: **Morche, Klaus, Dr.**
**Viktoriastrasse 12**
**D-6800 Mannheim 1(DE)**

(72) Erfinder: **Nützel, Karl, Dr.**
**Kornstrasse 23**
**D-6831 Neulussheim(DE)**

(72) Erfinder: **Schilling, Kurt**
**Friedrich-Ebert-Strasse 63**
**D-6830 Schwetzingen(DE)**

(74) Vertreter: **Gremm, Joachim, Dr. et al,**
**Bayer AG c/o Zentralbereich Patente, Marken und Lizenzen Bayerwerk**
**D-5090 Leverkusen(DE)**

## (54) Verfahren zur Herstellung von Polysulfiden.

(57) Verfahren zur Herstellung von Polysulfiden der allgemeinen Formeln (1), (2) und (3)

$$R-(S)_x-R \qquad (1)$$

$$HS-\left[(CH_2)_yS_x\right]_n-(CH_2)_y-SH \qquad (2)$$

$$R-\left[S_x-(CH_2)_y\right]_n-S_x-R \qquad (3)$$

in denen

R gleich oder verschieden einen gegebenenfalls durch Aryl-, Heteroaryl-, Hydroxyl-, Carboxyl- oder Alkcarboxy-Gruppen substituierten Alkylrest, einen gegebenenfalls durch Alkyl-, Halogen- oder Amino-Gruppen substituierten Arylrest und/oder einen Acylrest,

x Zahlen von 3 bis 5,

y Zahlen von 2 bis 10,

n Zahlen von 1 bis 20,

bedeuten, dadurch gekennzeichnet, daß man Mercaptane der Formeln (4) und/oder (5)

$$RSH \qquad (4)$$

$$HS-(CH_2)_y-SH \qquad (5)$$

in denen R und y die bereits angeführte Bedeutung besitzen mit Schwefel dehydrierend umsetzt.

EP 0 025 944 A1

0025944

Rhein-Chemie Rheinau GmbH
Patentabteilung

6800 Mannheim 81
E/Th

Verfahren zur Herstellung von Polysulfiden

Die Erfindung betrifft ein Verfahren zur Herstellung von Polysulfiden durch Dehydrierung entsprechender Merkaptane mittels Schwefel.

Polysulfide sowie mehrere Verfahren zu ihrer Herstellung sind bekannt.

Gemäß Seiten 210 - 228 aus Organic Sulfur Compounds (Autor: N. Karasch, Pergamon Press 1961) erfolgt ihre Herstellung durch direkte Schwefelung von Olefinen. Diese direkte Schwefelung verläuft bei höheren Temperaturen (170 - 180°C) und führt meist zu dunkel gefärbten Produkten mit relativ geringerem Schwefelgehalt (ca. 20 %). Die Produktzusammensetzung ist darüber hinaus uneinheitlich.

Aus Houben-Weyl Band 9, Seiten 83 - 92 (Georg Thieme-Verlag Stuttgart, 1955) ist die Umsetzung von Aromaten und Merkaptanen mit Chlorschwefel sowie die Umsetzung von Alkylhalogeniden mit Alkalipolysulfiden bekannt.

Weiterhin beschreibt die US-PS 2 549 525 die Reaktion von Olefinen mit Chlorschwefel und die anschließende Abspaltung des gebundenen Chlors mittels Natriumsulfid. Bei den Umsetzungen mit Schlorschwefel muß die freiwerdende Salzsäure gebunden bzw. das gebundene Chlor nachträglich abgespalten werden. Dies geschieht mit wäßrigen oder alkoholischen Alkalilaugen bzw. Alkalisulfidlösungen. Dadurch werden die Verfahren aufwendiger, vor allen werden aber die Volumenausbeuten stark gesenkt. Das Gleiche gilt für die Umsetzung von Alkylhalogeniden mit Alkalipolysulfiden.

Aufgabe der Erfindung ist es, ein chemisch eigenartiges Verfahren zur Herstellung von Polysulfiden bereitzustellen, welches die mit den bekannten Verfahren verbundenen Nachteile nicht aufweist.

Die Aufgabe konnte dadurch gelöst werden, daß man mono- und/oder mehrfachfunktionelle Merkaptane mit Schwefel dehydrierend - unter gleichzeitigem Einbau von Schwefel - umsetzt, wobei in hoher Ausbeute die entsprechenden Polysulfide entstehen.

Als allgemeine Reaktionsgleichungen gelten:

1) $RSH + S \longrightarrow R{-}(S)_x{-}R + H_2S$

2) $HS{-}(CH_2)_y{-}SH + S \longrightarrow HS{-}[(CH_2)_y\,S_x]_n{-}(CH_2)_y{-}SH + H_2S$

3) $RSH + HS{-}(CH_2)_y{-}SH + S \longrightarrow R{-}[S_x{-}(CH_2)_y]_n{-}S_x{-}R + H_2S$

Die dehydrierende Wirkung des Schwefels ist an sich bekannt. Die Hauptbedeutung der Dehydrierungsreaktion mit Schwefel liegt aber auf dem Gebiete der Konstitutionsaufklärung organischer Naturprodukte (A. Plattner, Die Chemie, 55, 131 (1942).

Ein Beispiel für eine präparative Anwendung ist die Dehydrierung von 3,4-Diphenylbenzo-cyclobuten - unter gleichzeitigem Einbau von Schwefel - zu 1,3-Diphenyliso-thiobenzofuran (M. Sauerbier, Chemiker Zeitung, 96, 1972).

Die Erfindung betrifft daher ein Verfahren zur Herstellung von Polysulfiden der allgemeinen Formeln (1), (2) und (3)

$$R-(S)_x-R \qquad (1)$$

$$HS-\left[(CH_2)_y S_x\right]_n-(CH_2)_y-SH \qquad (2)$$

$$R-\left[S_x-(CH_2)_y\right]_n-S_x-R \qquad (3)$$

in denen

R gleich oder verschieden einen gegebenenfalls durch Aryl-, Heteroaryl-, Hydroxyl-, Carboxyl- oder Alkcarboxy-Gruppen substituierten Alkylrest, einen gegebenenfalls durch Alkyl-, Halogen- oder Amino-Gruppen substituierten Arylrest, oder einen Acylrest,

x Zahlen von 3 bis 5, bevorzugt von 4 bis 5,

y Zahlen von 2 bis 10, bevorzugt von 2 bis 4,

n Zahlen von 1 bis 20, bevorzugt von 2 bis 10,

bedeuten, das dadurch gekennzeichnet ist, daß man Merkaptane der Formeln (4) und/oder (5)

RSH (4)

$HS-(CH_2)_y-SH$ (5)

in denen R und y die bereits angeführte Bedeutung besitzen mit Schwefel dehydrierend umsetzt.

In den allgemeinen Formeln (1) - (3) hat der Substituent R bevorzugt folgende Bedeutung:

$C_1$-$C_{16}$-Alkylrest gegebenenfalls substituiert durch Phenyl-, Naphthyl-, Anthryl-, Hydroxyl-, Carboxyl-, $C_1$-$C_{20}$-Alkcarboxy oder $C_1$-$C_8$-Alkthiocarboxy-Gruppen.

Arylreste wie Phenyl-, Naphthyl- oder Anthryl-Reste gegebenenfalls substituiert durch $C_1$-$C_{12}$-Alkyl, 1-5 Halogen oder 1-2 Amino-Gruppen.

Unter dem Begriff Arylreste werden auch durch Arylgruppen substituierte Arylreste verstanden.

$C_1$-$C_8$-Alkylacylreste sowie Benzoyl- und Naphthoyl-Rest.

Insbesondere bedeutet er:

$C_3$-$C_{12}$-Alkylrest oder Phenylrest.

Folgende Verbindungen seien namentlich angeführt:

Diethylpentasulfid, Di-isopropylpentasulfid, Di-isooctylpentasulfid, Diphenylpentasulfid, Di-4-chlorphenyl-pentasulfid, Diacetylpentasulfid, Dibenzoylpentasulfid, Didodecylpentasulfid, Di-p-toluylpentasulfid, Di-4-aminophenyl-pentasulfid.

Die als Ausgangsprodukt eingesetzten mono- und/oder polyfunktionellen Merkaptane sind bekannt. Wie aus dem eingangs beschriebenen Formelschema hervorgeht, hat der Substituent R die gleiche Bedeutung wie im Polysulfid der Formeln (1) - (3).

Beispielsweise können eingesetzt werden:

Ethylmerkaptan, iso-Propylmerkaptan, tert.-Butylmerkaptan, iso-Octylmerkaptan, iso-Dodecylmerkaptan, Thiophenol, p-Chlorthiophenol, 2-Aminothiophenol, p-Methylthiophenol, Dithioglykol, Pentaeryltrol-tetra-3-merkaptopropionat Merkaptoessigsäure.

Das Mengenverhältnis von Schwefel zu Merkaptan ist beliebig. Werden geringe Mengen an Schwefel eingesetzt,

bleibt viel Merkaptan unumgesetzt und es entstehen Trennungsschwierigkeiten. Werden zuviel Mengen Schwefel zugesetzt, fällt Schwefel nach der Umsetzung wieder aus. Als allgemein günstig hat sich einen Mengenverhältnis von 3,5-4 Mol Schwefel, insbesondere von 3,9 Mol Schwefel pro 2 Mol Merkaptan herausgestellt.

Die Umsetzungstemperatur ist nicht kritisch. Als bevorzugt sei ein Bereich von 20 bis 180°C, insbesondere ein Bereich von 80 bis 140°C erwähnt.

Durch Zugabe eines basischen Katalysators können die Reaktionstemperaturen erniedrigt und die Reaktionszeiten verkürzt werden. Es konnte ein Zusammenhang zwischen der Nucleophilie des Katalysators und seiner Wirksamkeit beobachtet werden: je größer die Nucleophilie desto besser ist auch die Wirksamkeit.

Durch Zugabe des Katalysators kann die Umsetzungstemperatur auf einen Bereich von 20-140°C, bevorzugt auf 80-100°C gesenkt werden.

Als Katalysatoren können prinzipiell alle basischen Verbindungen eingesetzt werden, bevorzugt primäre, sekundäre und tertiäre Alkyl-, Aryl- und Alryl-alkylamine, Alkoholate, Gemische von Wasser mit Metallhydroxiden und Amide.

Folgende Katalysatoren können eingesetzt werden:

Anilin, Cyclohexylamin, Gemisch aus $C_{12}$-$C_{14}$-Aminen (Primene 81 R, Röhn und Haas, Philadelphia), Di-cyclohexylamin, Diazadicyclo-[2,2,2]-octan (DABCO), Triethylamin, Triethylendiamin, tert.-Oktylamin, Distearylamin, Dimethylaminopyridin, N-(3-Aminopropyl)-morpholin, Morpholin, Pyridin, Ethanolamin, Diethanolamin, Triethanolamin, Caprolactam, Acetamid, $NaOCH_3$, $NaOC_2H_5$, K-tert.-butylat, $NaOH/H_2O$, $KOH/H_2O$, $CaO/H_2O$, $Ba(OH)_2 \cdot 8\ H_2O$.

Diese Katalysatoren können in Mengen von 0,1 bis 1 Gew.-%, bevorzugt von 0,1 bis 0,5 Gew.-%, insbesondere von 0,1 bis 0,2 Gew.-%, bezogen auf Merkaptan, eingesetzt werden.

Die nach dem erfindungsgemäßen Verfahren hergestellten Polysulfide können als Hochdruckzusätze zu Schmiermitteln, z.B. in Getriebeölen oder bei der Metallbearbeitung Verwendung finden.

Die Erfindung wird durch folgende Beispiele näher erläutert:

Beispiel 1

438 g iso-Oktylmercaptan werden mit 168 g Schwefel und 0,44 g Primene 81R (Gemisch aus $C_{12}$-$C_{14}$ Aminen Handelsprodukt der Firma Röhn und Haas, Philadelphia, USA) vermischt, unter Rühren auf 100°C aufgeheizt und drei Stunden bei dieser Temperatur gehalten. Dabei geht der Schwefel unter $H_2S$ - Entwicklung in Lösung. Anschließend wird eine Stunde mit Stickstoff ausgeblasen. Nach dem Abkühlen auf 70°C wird mit 1,76 g Primene 81R und 4,4 g $H_2O_2$ (30 %-ig) versetzt und 30 Minuten nachgerührt. Das so erhaltene Produkt wird bei 120°C im Wasserdampfstrom gestrippt. Nach Zusatz von 5 g Kieselgur wird abschließend filtriert.

Man erhält das Di-iso-oktylpentasulfid als hellgelbe, klare, ölige Flüssigkeit.

Ausbeute: 502,7 g = 90,5 % der Theorie
S-Gehalt: 41,6 %

Beispiel 2

110 g Thiophenol werden mit 56 g Schwefel vermischt und unter Rühren bei Raumtemperatur mit 0,11 g Primene 81R (Gemisch aus $C_{12}$-$C_{14}$ Aminen Handelsprodukt der Firma Röhn und Haas, Philadelphia, USA) versetzt. Nach Zugabe des Amins kommt die Reaktion unter $H_2S$ - Entwicklung sofort in Gang, wobei die Temperatur auf 10°C abfällt.

Nach Abklingen der Reaktion wird auf 70°C erwärmt und eine Stunde nachgerührt. Anschließend wird mit 1,5 g Primene 81R und 3 g $H_2O_2$ (30 %-ig) versetzt und eine halbe Stunde weitergerührt. Nach Abkühlen auf Raumtemperatur wird mit 2,3 g Kieselgur versetzt und filtriert.
Das Polysulfid fällt als gelbe, ölige Flüssigkeit an.

Ausbeute: 141,5 g = 95 % der Theorie
S-Gehalt: 48,7 %

Beispiel 3

90 g tert.-Butylmercaptan, 47 g Dithioglykol und 112 g Schwefel werden vermischt und unter Rühren bei Raumtemperatur langsam mit 0,7 g Primene 81 R( Gemisch aus $C_{12}$-$C_{14}$ Aminen Handelsprodukt der Firma Röhm und Haas, Philadelphia, USA) versetzt. Unter $H_2S$ - Entwicklung setzt eine heftige endotherme Reaktion ein, wobei die Temperatur auf 0°C absinkt. Innerhalb einer Stunde wird auf ca. 100°C erwärmt und drei Stunden bei dieser Temperatur gehalten. Nach Abkühlen auf 60-70°C wird mit 2,8 g $H_2O_2$ (30 %-ig) versetzt und 30 Minuten nachgerührt. Das Produkt wird bei 120°C im Wasserdampfstrom gestrippt, mit 1 % Kieselgur versetzt und filtriert. Man erhält das Polysulfid als klares, gelbes, dickflüssiges Öl.

Ausbeute: 210,7 g = 98 % der Theorie
S-Gehalt: 70,5 %

Beispiel 4

47 g Dithioglykol und 56 g Schwefel werden vermischt und unter Rühren bei Zimmertemperatur langsam mit 0,5 g Primene 81R(Gemisch aus $C_{12}$-$C_{14}$ Aminen, Handelsprodukt der Firma Röhn und Haas, Philadelphia, USA) versetzt. Unter $H_2S$ - Entwicklung setzt heftige endotherme Reaktion ein, wobei die Temperatur auf 0°C sinkt. Nach Abklingen der Reaktion wird auf 110-120°C aufgeheizt und zwei Stunden nachgerührt.

Das erkaltete Polysulfid fällt als gelbe, plastische Masse an.

Ausbeute: praktisch quantitativ
S-Gehalt 83,6 %

Patentansprüche

1. Verfahren zur Herstellung von Polysulfiden der allgemeinen Formeln (1), (2) und (3)

$$R-(S)_x-R \qquad (1)$$

$$HS-\left[(CH_2)_y S_x\right]_n-(CH_2)_y-SH \qquad (2)$$

$$R-\left[S_x-(CH_2)_y\right]_n-S_x-R \qquad (3)$$

in denen

R gleich oder verschieden einen gegebenenfalls durch Aryl-, Heteroaryl-, Hydroxyl-, Carboxyl- oder Alkcarboxy-Gruppen substituierten Alkylrest, einen gegebenenfalls durch Alkyl-, Halogen- oder Amino-Gruppen substituierten Arylrest und/oder einen Acylrest,

x Zahlen von 3 bis 5,

y Zahlen von 2 bis 10,

n Zahlen von 1 bis 20,

bedeuten, dadurch gekennzeichnet, daß man Mercaptane der Formeln (4) und/oder (5)

RSH (4)

$HS-(CH_2)_y-SH$ (5)

in denen R und y die bereits angeführte Bedeutung besitzen mit Schwefel dehydrierend umsetzt.

2. Verfahren nach Anspruch 1, dadurch gekennzeichnet, daß Mercaptane der Formeln (4) und (5) mit Schwefel dehydrierend umgesetzt werden, in denen R einen $C_3$-$C_{12}$ Alkylrest oder einen Phenylrest darstellt.

3. Verfahren nach Ansprüchen 1 und 2, dadurch gekennzeichnet, daß Mercaptane der Formeln (4) und (5) mit Schwefel dehydrierend umgesetzt werden, in denen y eine Zahl von 2 bis 4 darstellt.

4. Verfahren gemäß Ansprüchen 1 bis 3, dadurch gekennzeichnet, daß man die Umsetzung in Gegenwart eines basischen Katalysators durchführt.

5. Verfahren gemäß Anspruch 4, dadurch gekennzeichnet, daß der Katalysator in Mengen von 0,1 bis 1 Gew.-% bezogen auf das Mercaptan, eingesetzt wird.

6. Verfahren gemäß Ansprüchen 1 bis 5, dadurch gekennzeichnet, daß man pro 2 Mol Mercaptan 3,5-4 Mol Schwefel einsetzt.

7. Verfahren gemäß Ansprüchen 1 bis 6, dadurch gekennzeichnet, daß man pro 2 Mol Mercaptan 3,9 Mol Schwefel einsetzt.

8. Verfahren gemäß Ansprüchen 1 bis 3 und 6 bis 7, dadurch gekennzeichnet, daß man die Umsetzung bei einer Temperatur von 20 bis 180°C durchführt.

9. Verfahren gemäß Ansprüchen 4 und 5, dadurch gekennzeichnet, daß man die Umsetzung bei einer Temperatur von 20 bis 140°C durchführt.

0025944

Europäisches Patentamt

# EUROPÄISCHER RECHERCHENBERICHT

Nummer der Anmeldung
EP 80 10 5425

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 1 553 249 (MONSANTO CO.)<br>* Insgesamt *<br>-- | 1-9 |
| X | US - A - 2 237 625 (J.F. OLIN et al.)<br>* Insgesamt *<br>-- | 1-9 |
| X | US - A - 3 038 013 (P.F. WARNER et al.)<br>* Insgesamt *<br>-- | 1-9 |
| X | US - A - 3 022 351 (C.H. MIHM et al.)<br>* Insgesamt *<br>---- | 1-9 |

KLASSIFIKATION DER ANMELDUNG (Int. Cl.³)

C 07 C 149/12

RECHERCHIERTE SACHGEBIETE (Int Cl.³)

C 07 C 149/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 27-11-1980 | GRAMAGLIA |